# EUROPÄISCHE PATENTSCHRIFT

(11) **EP 3 468 504 B1**
(45) Veröffentlichungstag und Bekanntmachung des Hinweises auf die Patenterteilung: **08.07.2020**
(21) Anmeldenummer: 17734971.9
(22) Anmeldetag: 01.06.2017
(51) Int. Cl.: A61F 2/00, A61F 5/00

(54) **MEDIZINISCHE EINRICHTUNG ZUM ABSPERREN EINES KÖRPERKANALS**
MEDICAL ARRANGEMENT FOR SHUTTING OFF A BODY CHANNEL
DISPOSITIF MÉDICAL POUR BLOQUER UN CANAL CORPOREL

(30) Priorität: 14.06.2016 AT 2932016
(43) Veröffentlichungstag der Anmeldung: 17.04.2019
(73) Patentinhaber: A.M.I. Agency for Medical Innovations GmbH, 6800 Feldkirch (AT)
(72) Erfinder: HOHLRIEDER, Martin, 6840 Götzis (AT)
(74) Vertreter: Hofmann, Ralf U.
(86) Internationale Anmeldenummer: PCT/AT2017/000046
(87) Internationale Veröffentlichungsnummer: WO 2017/214644

(56) Entgegenhaltungen:
- DE-A1- 10 013 519
- FR-A1- 2 373 272
- US-A- 3 863 622
- US-A- 4 784 660
- US-A1- 2012 130 157

## Beschreibung

Die vorliegende Erfindung betrifft eine medizinische Einrichtung zum Absperren eines Körperkanals, umfassend ein Bandteil, das um das den Körperkanal umgebende Körpergewebe legbar ist und zu einem eine Durchtrittsöffnung für das Körpergewebe umschließenden Ring schließbar ist und das eine Hohlkammer aufweist, die einen Teil eines Arbeitsfluid-Aufnahmeraums der Einrichtung zur Aufnahme von Arbeitsfluid darstellt, und eine Pumpeinheit zum Fördern des Arbeitsfluids, wobei durch Einbringen des Arbeitsfluids in die Hohlkammer die Durchtrittsöffnung verkleinerbar ist, und einen Speicherbehälter mit einer eine Speicherkammer begrenzenden flexiblen Wand, wobei zum Aufbringen einer Zusatzkraft auf das durch die Durchtrittsöffnung geführte Körpergewebe das Volumen der Speicherkammer durch eine Erhöhung eines auf den Speicherbehälter einwirkenden Umgebungsdrucks verkleinerbar ist, und die Speicherkammer einen Teil eines Hilfsfluid-Aufnahmeraums der Einrichtung zur Aufnahme eines vom Arbeitsfluid getrennten Hilfsfluids bildet und der Hilfsfluid-Aufnahmeraum im Weiteren eine Expansionskammer eines Expansionskörpers der Einrichtung umfasst, wobei die Expansionskammer zum Aufbringen der Zusatzkraft mit dem Hilfsfluid befüllbar ist.

Medizinische Einrichtungen zum Absperren eines Körperkanals werden unter anderem als künstliche (Schließ-)Muskeln zur Unterstützung oder beim Ersatz von geschwächten natürlichen Muskeln im menschlichen oder tierischen Körper eingesetzt. Beispiele für Einsatzbereiche derartiger Einrichtungen sind Analbänder zum Verschließen eines, gegebenenfalls künstlichen, Anus und künstliche Schließmuskel zur Behandlung von Inkontinenz zum Verschließen der Urethra (= Harnröhre). Weitere Einsatzgebiete sind z. B. Bänder zum Verschließen eines Gangs für Gallenflüssigkeit. Das Bandteil derartiger medizinischer Einrichtungen wird auch als Cuff, Manschette oder artifizieller Sphinkter bezeichnet.

Die Hohlkammer des Bandteils kann vom Benutzer bei Bedarf entleert werden, um die Querschnittsfläche der Durchtrittsöffnung zu vergrößern und im Körperkanal enthaltene Stoffe und/oder Flüssigkeiten passieren zu lassen. Z. B. bei der Anwendung als künstlicher Schließmuskel für die Urethra erfolgt häufig ein anschließendes automatischen Verschließen des Körperkanals durch Rückpumpen von Arbeitsfluid (eventuell über ein Drosselventil) in die Hohlkammer des Bandteils. Eine Pumpeinheit zum Pumpen von Arbeitsfluid wird bei einem solchen künstlichen Harnsphinkter bei männlichen Patienten üblicherweise in das Skrotum implantiert. Das Pumpen von Arbeitsfluid aus der Hohlkammer kann dann durch Druck auf einen flexiblen Teil der Pumpeinheit erfolgen. Das Rückpumpen von Arbeitsfluid in die Hohlkammer kann durch ein federelastisches Element der Pumpeinheit erfolgen. Die Durchtrittsöffnung der medizinischen Einrichtung kann häufig auch durch eine bewusste Handlung des Benutzers wieder verkleinert werden, also durch eine manuelle Betätigung der Pumpeinheit.

In der US 3,863,622 A ist eine medizinische Einrichtung mit einem Bandteil, einem Speicherbehälter und zwei elastisch verformbaren Pumpkörpern zum Öffnen bzw. Schließen des Bandteils gezeigt. Mittels Rückschlagventilen wird die Förderrichtung des Arbeitsfluids, das zum Vergrößern und Verkleinern der Durchtrittsöffnung des Bandteils mittels der Pumpkörper befördert wird, vorbestimmt. Aus der DE 100 13 519 A1 geht ebenfalls eine medizinische Einrichtung zum Absperren eines Körperkanals hervor.

Problematisch bei medizinischen Einrichtungen zum Verengen oder Absperren eines Körperkanals ist, dass diese aufgrund des von der Einrichtung ausgeübten Druckes auf das Körpergewebe eine Erosion des Körpergewebes hervorrufen können. Der Druck des Arbeitsfluids in der Hohlkammer wird deshalb in der Regel so gewählt, dass die Erosion von Körpergewebe möglichst gering gehalten werden kann und gleichzeitig noch ein zuverlässiges Absperren des Körperkanals erreicht wird.

Bei einer Anspannung der Bauchmuskeln, wie dies z. B. beim Treppensteigen, Heben von Lasten, Niesen, Husten, Lachen, meist unwillkürlich auftritt, erfolgt eine kurzzeitige intraabdominelle (= innerhalb des Bauchraums) Erhöhung des Körperinnendrucks. Dabei wirken die kurzzeitigen Druckspitzen insbesondere auf die im Bauchraum angeordneten inneren (Hohl-)Organe, z. B. Harnblase und Darm. Dadurch nimmt der Druck in den (Hohl-)Organen zu, was zu einem Austritt von Stoffen und/oder Flüssigkeit durch den vom Bandteil abgesperrten Abschnitt des Körperkanals führen kann. Diese Art der Inkontinenz wird auch als Belastungsinkontinenz oder Stressinkontinenz bezeichnet.

Aus der US 5,478,305 A geht eine medizinische Einrichtung zur Behandlung von Harn- oder Stuhlinkontinenz hervor. Das in dieser Schrift als Cuff bezeichnete Bandteil ist aus Silikon hergestellt. Durch die Befüllung des Cuffs mit Arbeitsfluid steigt der Druck im Hohlraum des Cuffs an, wobei die dadurch bewirkte Verschiebung des inneren Abschnitts des Cuffs in Richtung zur Längsmittelachse hin den Körperkanal verschließt. Zwischen zwei Abschnitten einer Arbeitsfluidleitung, welche eine Pumpe mit dem Cuff fluidleitend verbindet, ist ein Speicherbehälter in Form eines flexiblen Ballons (= "Stressballon") aus elastisch dehnbarem Silikon angeordnet. Die vom Stressballon gebildete Speicherkammer ist mit Arbeitsfluid befüllt. Eine kurzzeitige Erhöhung des Körperinnendrucks bewirkt eine Volumenverkleinerung der Speicherkammer des Stressballons, wodurch Arbeitsfluid in den Cuff verdrängt wird. Durch die kurzzeitige Erhöhung des Drucks des Arbeitsfluids kann der Körperkanal auch während eines Stressereignisses abgesperrt gehalten und eine Leckage verhindert werden. Verringert sich der Körperinnendruck anschließend wieder, so vergrößert sich das Volumen der Speicherkammer des Stressballons und der Druck des Arbeitsfluids nimmt ab, wobei Arbeitsfluid aus dem Cuff herausströmt. Wird der Druck des Arbeitsfluids im Cuff zum Schließen des Körperkanals erhöht, so erhöht sich auch das Volumen des Stressballons, sodass mittels der Pumpe eine entsprechend große Flüssigkeitsmenge verschoben werden muss.

Aus der US 4,784,660 A geht eine Einrichtung der eingangs genannten Art hervor. Zusätzlich zu einem Arbeitsfluid-Aufnahmeraum enthält die Einrichtung einen elastisch dehnbaren Speicherbehälter mit einer Speicherkammer, die Teil eines Hilfsfluid-Aufnahmeraums ist. Mittels eines in einer Steuereinheit angeordneten Balgs können Druckerhöhungen des Hilfsfluids infolge eines Anstiegs des Körperinnendrucks zum Erzeugen einer Zusatzkraft auf das Arbeitsfluid übertragen werden, um einer Stressinkontinenz entgegenzuwirken.

Aufgabe der Erfindung ist es, eine vorteilhafte Einrichtung der eingangs genannten Art bereitzustellen, welche zur Behandlung von Stressinkontinenz eingesetzt werden kann.

Erfindungsgemäß gelingt dies durch eine Einrichtung mit den Merkmalen des Anspruchs 1.

Bei der medizinischen Einrichtung gemäß der Erfindung ist vorgesehen, dass die Wand des Speicherbehälters zumindest im Wesentlichen undehnbar ausgebildet ist.

Beim Befüllen des Speicherbehälters erfolgt also zumindest im Wesentlichen keine Materialdehnung der Wand, d. h. dass das Volumen der Speicherkammer zumindest im Wesentlichen nur durch Ausfalten der flexiblen Wand vergrößerbar ist. Das Volumen der Speicherkammer ist damit auf ein maximales Speichervolumen der Speicherkammer begrenzt.

Eine Ausbildung einer Wand als flexibel bedeutet, dass diese verbiegbar, insbesondere zusammenlegbar, ist.

Der Speicherbehälter ist im Körper, insbesondere im Bauchraum, implantierbar, wobei der im Körper wirkende Körperinnendruck auf den Speicherbehälter einwirkt. Beim Anspannen von Bauchmuskeln während eines Stressereignisses, ändert sich das Volumen der Speicherkammer des Speicherbehälters durch eine Erhöhung des Umgebungsdrucks (=Körperinnendruck). Während eines Stressereignisses liegt der auf den Speicherbehälter einwirkende Umgebungsdruck jedenfalls über dem Atmosphärendruck.

Als "im Wesentlichen undehnbar" wird die Materialeigenschaft der Wand des Speicherbehälters bezeichnet, bei welcher das Volumen der Speicherkammer ausgehend von einem maximal entfalteten Zustand bei einer Erhöhung des Drucks in der Speicherkammer im Betrieb der Einrichtung im Wesentlichen unverändert bleibt. Maßgebend sind hierbei die üblichen im Körperinnern wirkenden Drücke im Betrieb der medizinischen Einrichtung. Vorzugsweise ist vorgesehen, dass das Volumen der Speicherkammer ausgehend von einem maximal entfalteten Zustand des Speicherbehälters bei einer Erhöhung des Drucks in der Speicherkammer um 0,1 bar um weniger als 10%, vorzugsweise um weniger als 5%, zunimmt.

Die Wand des Speicherbehälters kann eine zumindest im Wesentlichen undehnbare Armierung aufweisen. Die Armierung kann z.B. netzartig ausgebildet sein. Günstigerweise weist die Armierung ein Elastizitätsmodul von zumindest 1000 N/mm², vorzugsweise von zumindest von 5000 N/mm² auf. Die Wand kann beispielsweise eine Matrix aus Silikon aufweisen, in welche die Armierung eingebettet ist.

Alternativ oder zusätzlich zu einer solchen zumindest im Wesentlichen undehnbaren Armierung kann die Wand des Speicherbehälters eine Lage aus einer zumindest im Wesentlichen undehnbaren Kunststofffolie aufweisen oder daraus bestehen. Die Kunststofffolie könnte z. B. aus Polyethylen, Polyamid oder ähnlichem hergestellt sein. Das Elastizitätsmodul der Kunststofffolie beträgt vorzugsweise zumindest 1000 N/mm², besonders bevorzugt zumindest 5000 N/mm². Insbesondere bei der Verwendung einer aus einer Kunststofffolie bestehenden Wand kann vorgesehen sein, dass die Wand biegeschlaff ausgebildet ist. D. h. dass die Wand nicht von selbst (= ohne Einwirkung einer äußeren Kraft) zu einer früheren Form zurückkehrt. Die biegeschlaffe Wand könnte auch als formlabil bezeichnet werden.

Bei der medizinischen Einrichtung gemäß der Erfindung ist vorgesehen, dass die Speicherkammer einen Teil eines Hilfsfluid-Aufnahmeraums der Einrichtung zur Aufnahme eines vom Arbeitsfluid getrennten Hilfsfluids bildet und der Hilfsfluid-Aufnahmeraum im Weiteren eine Expansionskammer eines Expansionskörpers der Einrichtung umfasst.

Das Hilfsfluid ist von dem Arbeitsfluid vollständig getrennt, d. h. dass kein Flüssigkeitsaustausch zwischen dem Hilfsfluid und dem Arbeitsfluid stattfindet.

Beim Auftreten eines Stressereignisses, z. B. hervorgerufen durch einen Anstieg des Körperinnendrucks bei einem Hustenanfall, erfolgt durch eine Verringerung des Volumens der Speicherkammer durch einen ausreichend großen von außen auf den Speicherbehälter einwirkenden Druck eine Vergrößerung der Expansionskammer des Expansionskörpers. Die Vergrößerung des Volumens des Expansionskörpers durch Einfüllen von Hilfsfluid in die Expansionskammer erfolgt insbesondere durch Dehnen oder Entfalten des Expansionskörpers. Hierzu kann vorgesehen sein, dass der Expansionskörper elastisch dehnbar ist. In anderen Ausführungsformen kann der Expansionskörper aber auch aus einem zumindest im Wesentlichen undehnbaren Material ausgebildet sein.

In einer möglichen Variante der Erfindung kann vorgesehen sein, dass der Expansionskörper im Arbeitsfluid-Aufnahmeraum angeordnet ist. Beim Befüllen der Expansionskammer mit Hilfsfluid und der dadurch bewirkten Volumenvergrößerung der Expansionskammer erfolgt eine Erhöhung des Drucks des Arbeitsfluids, wobei der Innenabschnitt des Bandteils weiter in Richtung zur Längsmittelachse der Durchtrittsöffnung verschoben wird. Es kommt dadurch zu einer stärkeren Komprimierung des den Körperkanal umgebenden Körpergewebes. Der Expansionskörper könnte beispielsweise in der Hohlkammer des Bandteils oder der Arbeitsfluidleitung angeordnet sein. Besonders bevorzugt ist der Expansionskörper in der Pumpeinheit angeordnet.

In einer anderen möglichen Variante der Erfindung kann vorgesehen sein, dass der Expansionskörper an einer der Durchtrittsöffnung zugewandten Seite des Bandteils am Bandteil angeordnet ist, also am Innenabschnitt des Bandteils. Ein Befüllen der Expansionskammer mit Hilfsfluid bewirkt eine Vergrößerung des Volumens der Expansionskammer, welche direkt komprimierend auf das den Körperkanal umgebende Körpergewebe einwirkt. Dadurch kann ein zuverlässiges Absperren des Körperkanals während eines Stressereignisses zu gewährleistet werden.

Weitere Vorteile und Einzelheiten der Erfindung werden im Folgenden anhand der beiliegenden Zeichnungen erläutert. In diesen zeigen:
Fig. 1 eine schematische Darstellung eines ersten Ausführungsbeispiels einer als künstlicher Urethrasphinkter ausgebildeten medizinischen Einrichtung, in einem Freigabezustand des Bandteils, in welchem der Harnleiter geöffnet ist;
Fig. 2 eine Darstellung analog Fig. 1 in einem Absperrzustand des Bandteils, in welchem der Harnleiter geschlossen ist;
Fig. 3 eine Darstellung analog Fig. 1 in einem Stresszustand des Bandteils, in welchem eine Zusatzkraft auf den Harnleiter ausgeübt wird;
Fig. 4 und 5 Schnittdarstellungen des Speicherbehälters entsprechend den Fig. 2 und 3;
Fig. 6 und 7 Schrägsichten des freien, also nicht implantierten, Bandteils der Einrichtung in einem geöffneten und geschlossenen Zustand, und zwar entsprechend dem Freigabezustand;
Fig. 8 einen Längsmittelschnitt (parallel zur Längsmittelachse der Durchtrittsöffnung und diese durchsetzend) durch das Bandteil im Zustand gemäß Fig. 7;
Fig. 9 Einen Längsmittelschnitt analog Fig. 8, aber im Absperrzustand des Bandteils;
Fig. 10 eine schematische Darstellung einer Ausführungsform eines künstlichen Urethrasphinkters in einem Absperrzustand des Bandteils, in welchem der Harnleiter geschlossen ist;
Fig. 11 eine Darstellung analog Fig. 10 in einem Stresszustand des Bandteils, in welchem eine Zusatzkraft auf den Harnleiter ausgeübt wird;
Fig. 12 und Fig. 13 schematische Darstellungen eines ersten Ausführungsbeispiels gemäß der Erfindung analog zu den Fig. 10 und 11, und
Fig. 14 und 15 schematische Darstellungen eines zweiten erfindungsgemäßen Ausführungsbeispiels in einem Absperrzustand und einem Stresszustand des Bandteils.

Ein Bandteil 1 der medizinischen Einrichtung ist ringförmig um das den Körperkanal umgebende Körpergewebe 2, hier die Harnröhre, legbar. Das Bandteil 1 weist eine Hohlkammer 3 auf, die sich in Richtung der Längserstreckung des Bandteils 1 erstreckt, in den Ausführungsbeispielen im Wesentlichen über die gesamte Länge des Bandteils 1. Das Bandteil 1 ist somit schlauchartig ausgebildet mit beidseitig verschlossenen Enden.

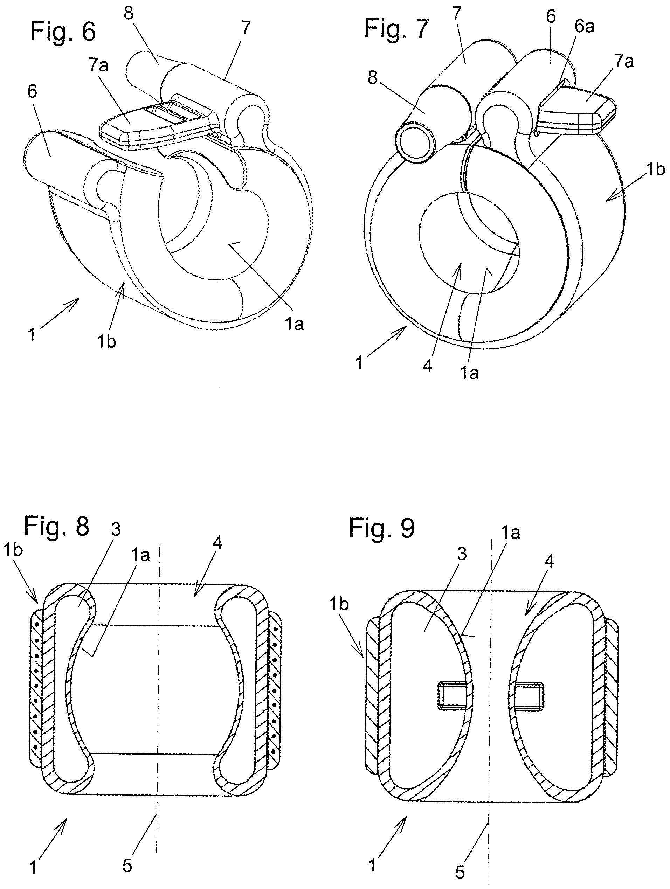

An den beiden Enden des Bandteils 1 sind ein erstes und ein zweites Verschlussteil 6, 7 angeordnet. Das erste Verschlussteil 6 besitzt eine Einstecköffnung 6a, in welche eine Zunge 7a des zweiten Verschlussteils 7 einsteckbar und darin verrastbar ist, vgl. Fig. 6.

Die Verschlussteile 6, 7 bilden somit einen Verschluss, mit welchem das Bandteil 1 zu einem Ring, insbesondere Kreisring, geschlossen werden kann, vgl. Fig. 7. Im geschlossenen Zustand umschließt das Bandteil 1 eine Durchtrittsöffnung 4 für das den Körperkanal umgebende Körpergewebe 2.

In der Hohlkammer 3 befindet sich ein Arbeitsfluid, insbesondere eine Flüssigkeit, z.B. Kochsalzlösung. Die Größe der Durchtrittsöffnung 4 hängt von der Menge des Arbeitsfluids in der Hohlkammer 3 ab. Durch Einbringen von Arbeitsfluid in die Hohlkammer 3 kann die Durchtrittsöffnung 4 verkleinert werden. Hierbei wird ein flexibler Innenabschnitt 1a des Bandteils 1, welcher zur Längsmittelachse 5 der Durchtrittsöffnung 4 benachbart ist, in Richtung zur Längsmittelachse 5 verschoben, wie dies bekannt ist. Durch Abführen von Arbeitsfluid aus der Hohlkammer 3 kann die Durchtrittsöffnung 4 wieder vergrößert werden.

Fig. 8 zeigt den Zustand, in welchem die Durchtrittsöffnung 4 am größten ist (wobei der Druck des Arbeitsfluids in der Hohlkammer 3 dem Umgebungsdruck entspricht). Fig. 9 zeigt einen mit dem Arbeitsfluid befüllten Zustand, insbesondere dem maximal mit Arbeitsfluid befülltem Zustand (wobei der Druck des Arbeitsfluids in der Hohlkammer 3 über dem Umgebungsdruck liegt). In Fig. 9 sind Faltenbildungen, wie sie sich insbesondere ergeben würden, wenn das Bandteil nicht um den Harnleiter gelegt ist, nicht dargestellt. Ein von der Längsmittelachse 5 abgelegener Rückenabschnitt 1b des Bandteils 1 kann gegenüber dem Innenabschnitt 1a steif ausgebildet sein, insbesondere mittels einer Verstärkungslage, wodurch eine Verformung des Rückenabschnitts 1b zumindest weitgehend vermieden werden kann.

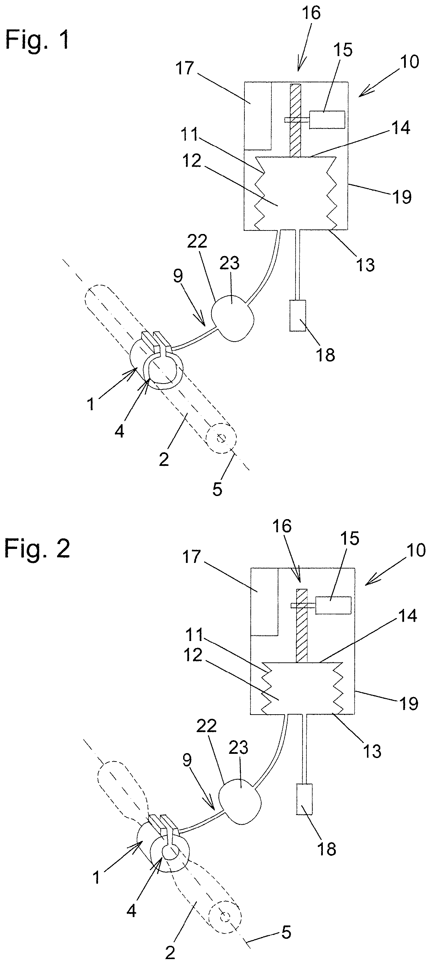

Im geschlossenen Zustand des um den Körperkanal gelegten Bandteils 1 kann dieses also einen Freigabezustand, in welchem der Körperkanal geöffnet ist (vgl. Fig. 1), und einen Absperrzustand, in welchem der Körperkanal geschlossen ist (vgl. Fig. 2), einnehmen. Im Freigabezustand kann der Druck des Arbeitsfluids in der Hohlkammer 3 beispielsweise dem Atmosphärendruck entsprechen. Im Absperrzustand wird die Hohlkammer 3 mit einer solchen Menge von Arbeitsfluid befüllt, dass der Körperkanal geschlossen ist.

Unterschiedliche Modifikationen der Ausbildung des Bandteils sind denkbar und möglich, so wäre es beispielsweise möglich, spezielle am Bandteil 1 angebrachte Verschlussteile überhaupt wegzulassen und die beiden Enden des Bandteils miteinander zu vernähen.

Das Bandteil 1 kann in bekannter Weise aus Silikon bestehen. Auch andere körperverträgliche Materialien sind grundsätzlich einsetzbar.

Im Ausführungsbeispiel ist an einem der Verschlussteile ein Anschlussstutzen 8 angeformt, dessen Hohlraum über einen durch das Verschlussteil 7 laufenden Kanal mit der Hohlkammer 3 in Verbindung steht. Ein solcher Anschlussstutzen könnte auch an einer anderen Stelle des Bandteils vorgesehen sein. Eine als Schlauch ausgebildete Arbeitsfluidleitung 9 ist am Anschlussstutzen 8 angeschlossen.

Der durch das Verschlussteil 7 laufende Kanal und die Hohlkammer 3 bilden jeweils einen Teil eines Arbeitsfluid-Aufnahmeraums zur Aufnahme von Arbeitsfluid. Der Arbeitsfluid-Aufnahmeraum der medizinischen Einrichtung umfasst den gesamten zusammenhängenden Innenhohlraum der Einrichtung, in welchem sich im Betrieb Arbeitsfluid befindet. Auch der innere Kanal der Arbeitsfluidleitung 9 und ein im Betrieb der Einrichtung mit Arbeitsfluid befüllter Innenraum 12 eines Pumpteils 11 bilden jeweils einen Teil des Arbeitsfluid-Aufnahmeraums für das Arbeitsfluid. Das Bandteil 1 steht über die Arbeitsfluidleitung 9 mit dem vom Bandteil 1 örtlich getrennten Pumpteil 11 der Pumpeinheit 10 in Verbindung, vgl. Fig. 1 bis 3. Mittels der Pumpeinheit 10 kann die Menge von Arbeitsfluid in der Hohlkammer 3 des Bandteils 1 verändert werden.

In den Ausführungsbeispielen wird das Pumpteil 11 von einem Balg gebildet, der von einem Bodenteil 13 und einem Deckelteil, welches ein Stellelement 14 darstellt, verschlossen ist. Ein elektrischer Antrieb 15 wirkt über ein Getriebe 16, beispielsweise einen Gewindetrieb, auf das Stellelement 14 ein, um das Volumen des Innenraums 12 zu ändern. Das Getriebe 16 ist günstigerweise selbsthemmend ausgebildet, sodass eine einmal eingestellte Position des Stellelements 14 ohne Zufuhr von elektrischer Energie an den Antrieb 15 gehalten wird.

Das Pumpteil 11 bildet im Ausführungsbeispiel also gleichzeitig ein Reservoir für das Arbeitsfluid, mit welchem die Hohlkammer 3 des Bandteils 1 zum Schließen des Körperkanals befüllt wird. Das Pumpteil 11 könnte beispielsweise auch von einer Kolben-Zylinder-Einheit gebildet werden, wobei das Stellelement 14 vom Kolben dieser Kolben-Zylinder-Einheit gebildet würde.

Der elektrische Antrieb 15 wird von einer Steuerelektronik 17 der Pumpeinheit 10 angesteuert, welche auch eine nicht dargestellte Batterie zur Speisung des Antriebs 15 mit elektrischem Strom aufweist. Die Bedienung der Steuerelektronik 17 durch den Benutzer erfolgt über eine geeignete, nicht näher dargestellte Benutzerschnittstelle. Bei der Benutzerschnittstelle kann es sich um eine per Kabel oder über Funk mit der Steuerelektronik 17 verbundene Bedieneinheit mit entsprechenden Schaltern handeln.

Die Benutzerschnittstelle kann außerhalb des Körpers angeordnet sein. Eine Implantation der Benutzerschnittstelle ist denkbar und möglich. Eine separate Benutzerschnittstelle könnte grundsätzlich auch entfallen, wobei mindestens ein vom Benutzer betätigbares Bedienelement an der Pumpeinheit 10 anzuordnen wäre. Dieses müsste entsprechend von außerhalb des Körpers betätigbar sein.

Die Komponenten der Pumpeinheit 10 sind in einem Gehäuse 19 angeordnet. Das Gehäuse 19 besteht aus einem körperverträglichen Material oder ist von einem solchen umhüllt.

Zum Befüllen des Arbeitsfluid-Aufnahmeraums der medizinischen Einrichtung mit Arbeitsfluid ist in herkömmlicher Weise ein Port 18 vorhanden. Dieser kann beispielsweise über einen Schlauch an das Pumpteil 11 angeschlossen sein.

Die medizinische Einrichtung weist im Weiteren einen Speicherbehälter 22 mit einer Speicherkammer 23 auf.

Der Speicherbehälter 22 ist bei der in den Figuren 1 bis 5 gezeigten medizinischen Einrichtung zwischen zwei Abschnitten 9', 9" der Arbeitsfluidleitung 9, welche die Hohlkammer 3 des Bandteils 1 mit dem Innenraum 12 des Pumpteils 11 verbindet, angeordnet. Im Betrieb der Einrichtung ist die Speicherkammer 23 mit Arbeitsfluid befüllt und bildet somit einen Teil des Arbeitsfluid-Aufnahmeraums der Einrichtung.

Die Speicherkammer 23 des Speicherbehälters 22 ist durch eine flexible Wand 22a begrenzt. Diese ist zumindest im Wesentlichen undehnbar ausgebildet. D.h., dass sich das Volumen der Speicherkammer 23 beim Betrieb der Einrichtung ausgehend von einem maximal entfalteten Zustand des Speicherbehälters 22 bei einer Erhöhung des Drucks in der Speicherkammer 23 zumindest im Wesentlichen nicht erhöht. Der Druck in der Speicherkammer 23 entspricht dem Druck des Arbeitsfluids.

Eine Erhöhung des Drucks in der Speicherkammer 23 (= Druck des Arbeitsfluids) des maximal entfalteten Speicherbehälters 22 um 0,1 bar (ausgehend vom Druck der im Absperrzustand des Bandteils 1 vorliegt) führt höchstens zu einer Zunahme des Volumens der Speicherkammer 23 um weniger als 5%.

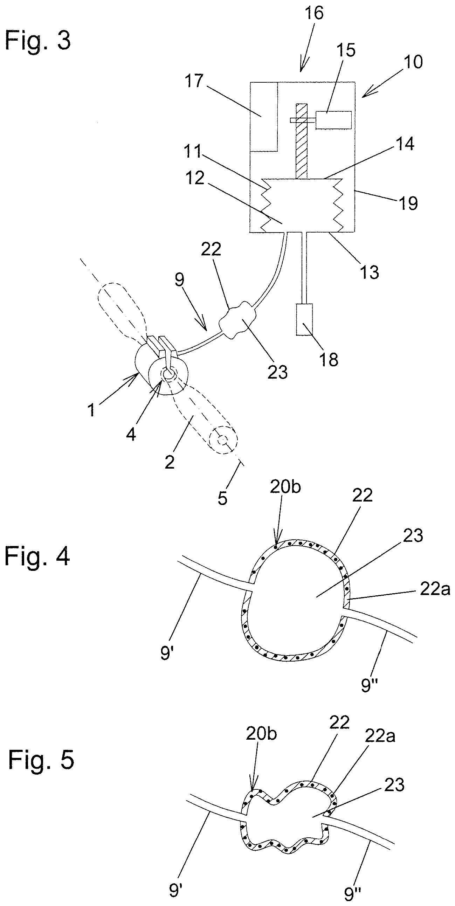

Die Wand 22a des Speicherbehälters 22 weist eine zumindest im Wesentlichen undehnbare Armierung 22b auf. Die Armierung 22b weist günstigerweise ein Elastizitätsmodul von zumindest 1.000 N/mm², vorzugsweise von zumindest 5.000 N/mm² auf. Die Armierung 22b ist in ein Grundmaterial (=Matrix) der Wand 22a eingebettet, vgl. Fig. 4 und 5. Das Grundmaterial der Wand 22a besteht aus Silikon.

Die Armierung 22b ist netzartig ausgebildet, d.h. dass einzelne Fasern oder Stränge der Armierung an Kreuzungspunkten verknüpft sind rechteckige oder rautenförmige Maschen der Armierung 22b bilden. Die netzartig ausgebildete Armierung 22b könnte auch als Geflecht bezeichnet werden.

Der Speicherbehälter 22 ist zusammenfaltbar, wobei das Volumen der Speicherkammer 23 durch ein Zusammenfalten oder Auseinanderfalten des Speicherbehälters 22 veränderbar ist, vgl. Fig. 4 und 5. Ein Zusammenfalten kann insbesondere durch eine Erhöhung des Umgebungsdrucks (=Körperinnendruck) auf einen über dem Druck des Arbeitsfluid liegenden Druck erfolgen.

Die Wand 22a des Speicherbehälters 22 könnte alternativ oder zusätzlich zur Armierung 22b eine zumindest im Wesentlichen undehnbare Kunststofffolie aufweisen oder von einer zumindest im Wesentlichen undehnbaren Kunststofffolie gebildet sein. Bei einer Ausbildung der Wand 22a aus einer Kunststofffolie könnte vorgesehen sein, dass die Wand biegeschlaff ausgebildet ist, d.h. die Wand 22a kehrt dann nicht von selbst zu einer früheren Form zurück. Die biegeschlaff ausgebildete Wand 22a könnte auch als formlabil bezeichnet werden.

Günstigerweise weist die Kunststofffolie ein Elastizitätsmodul von zumindest 1.000 N/mm², vorzugsweise von zumindest 5.000 N/mm² auf.

Die Kunststofffolie könnte z.B. aus Polyethylen oder Polyamid gebildet sein.

Im in Fig. 1 dargestellten Freigabezustand und im in Fig. 2 dargestellten Absperrzustand des Bandteils 1 entspricht das Volumen der Speicherkammer 23 einem maximalen Wert des Volumens (=Speichervolumen). Das Volumen der Speicherkammer 23 ändert sich hier somit zwischen dem Freigabezustand und dem Absperrzustand des Bandteils 1 nicht (solange nicht von außen ein zusätzlicher Druck auf den Speicherbehälter 22 einwirkt, vgl. die Beschreibung weiter unten). Es könnte aber auch vorgesehen sein, dass das Volumen der Speicherkammer 23 im Freigabezustand des Bandteils 1 kleiner als das maximale Speichervolumen der Speicherkammer 23 ist, d.h. dass der Speicherbehälter 22 dann nicht vollständig entfaltet ist.

Steigt der Druck im Körperinneren (=Körperinnendruck) an, z.B. aufgrund eines Hustenanfalls, so wirkt eine durch den Körperinnendruck hervorgerufene Kraft direkt auf den Speicherbehälter 22 ein. Ist im Absperrzustand des Bandteils 1 der Körperinnendruck größer als der Druck des Arbeitsfluids, so wird der Speicherbehälter 22 komprimiert. Dadurch erhöht sich der Drucks des Arbeitsfluids, wobei zusätzlich Arbeitsfluid in die Hohlkammer 3 des Bandteils 1 eingebracht wird. Der Innenabschnitt 1a des Bandteils 1 wird in Richtung hin zur Längsmittelachse verschoben und dabei eine Zusatzkraft auf das durch die Durchtrittsöffnung 4 geführte Körpergewebe 2 aufgebracht. Dieser Zustand des Bandteils 1 wird in dieser Schrift als Stresszustand des Bandteils 1 bezeichnet und ist in Fig. 3 dargestellt.

Verringert sich in der Folge der Körperinnendruck und damit der Druck des Arbeitsfluids, so vergrößert sich das Volumen der Speicherkammer 23 des Speicherbehälters 22 unter Aufnahme von Arbeitsfluid. Nach dem Stressereignis befindet sich das Bandteil 1 somit wiederum im Absperrzustand, vgl. Fig. 2.

Da die Wand 22a des Speicherbehälters 22 zumindest im Wesentlichen undehnbar ausgebildet ist, übt der Speicherbehälter 22 bei einer Befüllung der Speicherkammer 23 unter Vergrößerung des Volumens (ausgehend von einem zusammengefalteten Zustand des Speicherbehälters 22) zumindest im Wesentlichen keine elastische Rückstellkraft auf den Inhalt der Speicherkammer, der im ersten Ausführungsbeispiel Arbeitsfluid ist, aus. Wenn der Körperkanal ausgehend vom Absperrzustand des Bandteils 1, z.B. zum Ablassen von Urin, geöffnet werden soll, d.h. das Bandteil 1 durch Verlagern von Arbeitsfluid aus der Hohlkammer 3 in den Innenraum 12 des Pumpteils 11 den Freigabezustand (siehe z.B. Fig. 1) einnimmt und auch kein Stressereignis vorliegt, bleibt die Volumenänderung der Speicherkammer 23 im Wesentlichen gleich Null oder relativ gering. Beim Verstellen des Bandteils 1 vom Absperrzustand in den Freigabezustand und umgekehrt muss somit kein oder nur wenig Arbeitsfluid aus dem und in den Speicherbehälter 22 gefördert werden. Die Menge an zu verschiebendem Arbeitsfluid kann dadurch minimiert und der damit verbundene Energieaufwand zum Verschieben von Arbeitsfluid vermindert werden. Das Intervall zum Aufladen oder Austauschen der Batterie der Steuerelektronik 17 kann dadurch verlängert werden.

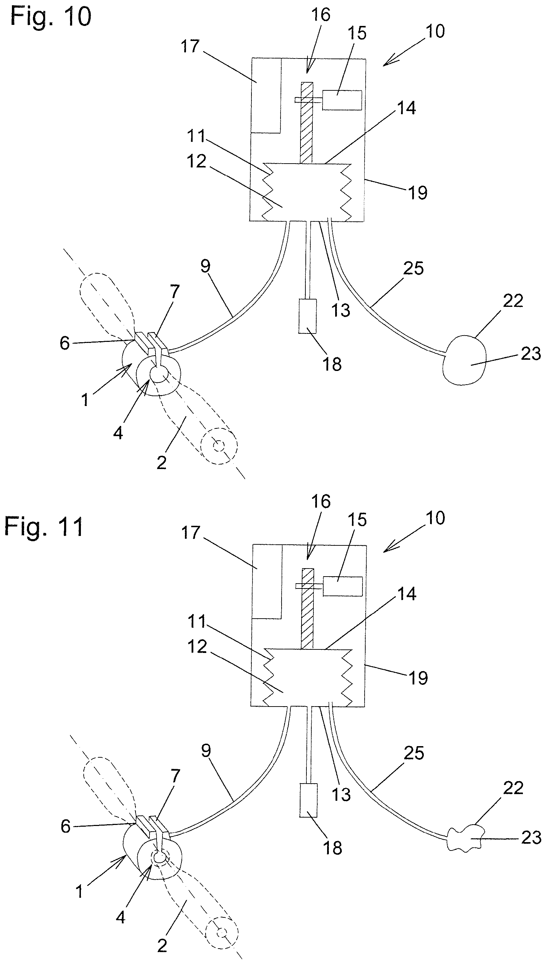

In den Fig. 10 und 11 ist eine Variante einer medizinischen Einrichtung dargestellt. Der Aufbau der Pumpeinheit 10 und des Bandteils 1 entspricht jenem der in den Figuren 1 bis 9 dargestellten medizinischen Einrichtung, sodass in den folgenden Erläuterungen hauptsächlich auf die Unterschiede zur zuvor erläuterten medizinischen Einrichtung hingewiesen wird. Abgesehen von den im Folgenden angeführten Unterschieden gelten die Erläuterungen zu der in den Figuren 1 bis 9 dargestellten medizinischen Einrichtung auch für die in den Figuren 10 und 11 dargestellte medizinische Einrichtung. So wird u.a. hinsichtlich der Ausbildung der Wand 22a des Speicherbehälters 22 und hinsichtlich der in den Fig. 10 und 11 nur schematisch dargestellten Verschlussteile 6, 7 des Bandteils 1 auf die Erläuterungen zu der in den Figuren 1 bis 9 dargestellten medizinischen Einrichtung verwiesen.

Bei der medizinischen Einrichtung gemäß der Fig. 10 und 11 ist vorgesehen, dass der Speicherbehälter 22 über eine separate Verbindungsleitung 25 mit der Pumpeinheit 10 verbunden ist. Die Speicherkammer 23 des Speicherbehälters 22 bildet einen Teil des Arbeitsfluid-Aufnahmeraums der Einrichtung. Der Speicherbehälter 22 weist nur einen Anschluss für Arbeitsfluid auf und ist ansonsten, vorzugsweise vollständig, geschlossen ausgebildet. Während eines Stressereignisses erfolgt somit ein Verschieben von Arbeitsfluid aus der Speicherkammer 23 über den inneren Kanal der Verbindungsleitung 25 in den Innenraum 12 des Pumpteils 11. Die Verbindungsleitung 25 ist als Schlauch ausgeführt.

Ansonsten verhält sich die medizinische Einrichtung beim Auftreten eines Stressereignisses analog zur Einrichtung gemäß der Fig. 1 bis 9, weshalb auf die entsprechenden Erläuterungen zur in den Fig. 1 bis 9 dargestellten medizinischen Einrichtung verwiesen wird.

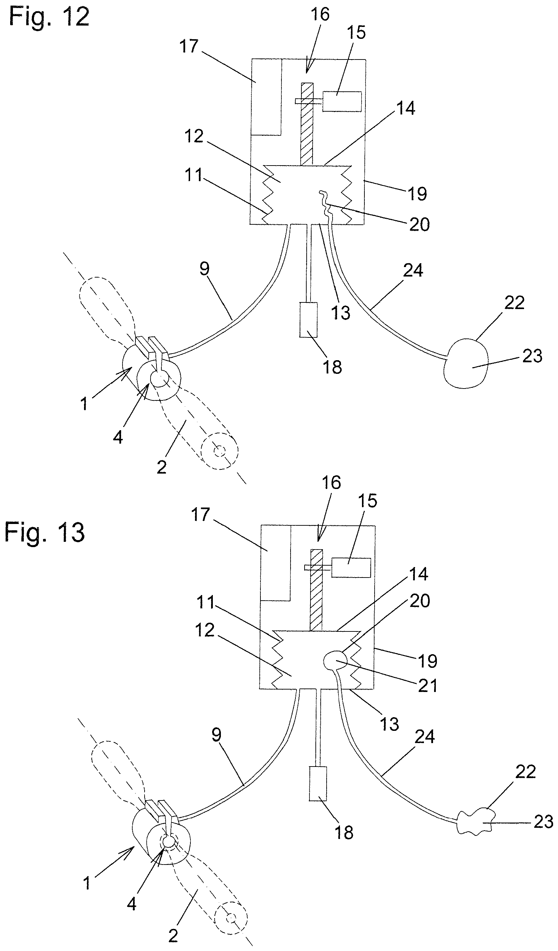

In den Fig. 12 und 13 ist ein erstes Ausführungsbeispiel einer medizinischen Einrichtung gemäß der Erfindung dargestellt. Der Aufbau der Pumpeinheit 10 und des Bandteils 1 entspricht jenen der in den Fig. 1 bis 9 und Fig. 10 und 11 dargestellten medizinischen Einrichtungen. Der Aufbau des Speicherbehälters 22 entspricht jenem der in Fig. 10 und 11 dargestellten medizinischen Einrichtung, sodass in den Erläuterungen zum ersten Ausführungsbeispiel hauptsächlich auf die Unterschiede zu den in den Figuren 1 bis 9 und 10, 11 dargestellten Einrichtungen hingewiesen wird. Abgesehen von den im Folgenden angeführten Unterschieden gelten die Erläuterungen zu den in den Fig. 1 bis 9 und 10 und 11 dargestellten Einrichtungen somit auch für das erste Ausführungsbeispiel gemäß der Erfindung.

Bei der medizinischen Einrichtung gemäß dem ersten erfindungsgemäßen Ausführungsbeispiel ist vorgesehen, dass die Speicherkammer 23 des Hilfsfluidbehälters 22 mittels einer Hilfsfluidleitung 24 mit einer Expansionskammer 21 eines Expansionskörpers 20 fluidleitend verbunden ist. Das vom Arbeitsfluid getrennte Hilfsfluid könnte z.B. über einen nicht näher dargestellten Port in die Hilfsfluidleitung 24 oder direkt in die Speicherkammer 23 eingebracht werden. Beim Hilfsfluid handelt es sich günstigerweise um eine Flüssigkeit, z.B. Kochsalzlösung.

Die Speicherkammer 23 bildet bei diesem Ausführungsbeispiel somit einen Teil eines Hilfsfluid-Aufnahmeraums der Einrichtung zur Aufnahme eines vom Arbeitsfluid getrennten Hilfsfluids. Auch die Expansionskammer und der innere Kanal der Hilfsfluidleitung 24 bilden einen Teil des Hilfsfluid-Aufnahmeraums der Einrichtung.

Der Expansionskörper 20 ist im ersten Ausführungsbeispiel im mit Arbeitsfluid gefüllten Arbeitsfluid-Aufnahmeraum der Einrichtung angeordnet, nämlich im Innenraum 12 des Pumpteils 11, vgl. Fig. 12 und 13. D.h. der Expansionskörper 20 ist von Arbeitsfluid umgeben.

Das Volumen der Expansionskammer 21 ist durch Einbringen von Hilfsfluid in die Expansionskammer 21 durch Entfalten oder Dehnen des Expansionskörpers 20, vergrößerbar. Wenn die Vergrößerung rein durch Entfalten erfolgt, kann der Expansionskörper 20 aus einem undehnbaren Material bestehen, sodass das maximale Volumen der Expansionskammer 21 begrenzt ist. Somit ist eine Druckerhöhung des Arbeitsfluids durch die Begrenzung des Volumens der Expansionskammer 21 begrenzt. Andererseits kann das Volumen der Expansionskammer 21 durch eine elastisch dehnbare Ausbildung des Expansionskörpers 20 vergrößerbar sein.

Der Expansionskörper 20 kann auch biegeschlaff ausgebildet sein, der Expansionskörper 20 ist dann formlabil, wie dies in Fig. 12 angedeutet ist.

Zumindest in dem in Fig. 12 dargestellten Absperrzustand des Bandteils 1 steht das Hilfsfluid unter einem geringeren Druck als das Arbeitsfluid. Die Expansionskammer 21 des Expansionskörpers ist vorzugsweise vollständig zusammengefaltet. Damit befindet sich zumindest im Wesentlichen kein Hilfsfluid in der Expansionskammer 21. Das Volumen der Expansionskammer 21 ist somit zumindest im Wesentlichen gleich Null. Dies könnte in einer modifizierten Ausführungsform grundsätzlich auch anders sein.

Auch im nicht näher dargestellten Freigabezustand des Bandteils 1 könnte die Expansionskammer 21 vollständig zusammengefaltet sein, also mit einem Volumen von im Wesentlichen gleich Null. Dies ist dann der Fall, wenn das Hilfsfluid unter einem geringeren Druck als das Arbeitsfluid steht und/oder wenn das Hilfsfluid bei Gleichheit des Drucks (des Hilfsfluids und des Arbeitsfluids) durch die Elastizität des Expansionskörpers 20 aus der Expansionskammer 21 heraus verdrängt wird. Auch ein gewisses Restvolumen der Expansionskammer 21 im Freigabezustand kann vorhanden sein.

Steigt nun der Druck im Körperinneren (=Körperinnendruck) an, beispielsweise bei einem Hustenanfall, so wirkt die durch den Körperinnendruck hervorgerufene Kraft direkt auf den Speicherbehälter 22 ein. Beim Ausüben einer Kraft auf den Speicherbehälter 22 erhöht sich der Druck des Hilfsfluids entsprechend. Ist der Körperinnendruck, und damit der Druck des Hilfsfluids, größer als der Druck des Arbeitsfluids, so wird der Speicherbehälter 22 komprimiert und Hilfsfluid wird aus der Speicherkammer 23 in die Expansionskammer 21 verdrängt. Falls der Expansionskörper 20 der Expansion der Expansionskammer 21 eine elastische Rückstellkraft entgegensetzt, kann auch diese durch den Druck des Hilfsfluids überwunden werden (Der Expansionskörper 20 wird somit erst expandiert, wenn der Umgebungsdruck den Druck des Arbeitsfluids so weit übersteigt, dass auch die elastische Rückstellkraft durch den Druck des Hilfsfluids überwunden wird). Die resultierende Vergrößerung des Volumens der Expansionskammer 21 führt dabei zu einer Erhöhung des Drucks im Innenraum 12 des Pumpteils 11. Dabei wird Arbeitsfluid aus dem Innenraum 12 ausgestoßen und in die Hohlkammer 3 des Bandteils eingebracht. Der Innenabschnitt 1a des Bandteils 1 wird somit in Richtung hin zur Längsmittelachse 5 verschoben und dabei die Zusatzkraft auf das durch die Durchtrittsöffnung 4 geführte Körpergewebe 2 aufgebracht. Dieser Zustand des Bandteils 1 (=Stresszustand) ist in Fig. 13 dargestellt.

Verringert sich in der Folge der Körperinnendruck und damit der Druck des Hilfsfluids wieder, sodass der Druck des Hilfsfluids kleiner als der Druck des Arbeitsfluid ist (gegebenenfalls zuzüglich der elastischen Rückstellkraft des Expansionskörpers 20), so vergrößert sich das Volumen der Speicherkammer 23 des Speicherbehälters 22 unter Aufnahme von aus der Expansionskammer 21 des Expansionskörpers 20 abgeführtem Hilfsfluid. Nach dem Stressereignis befindet sich das Bandteil 1 wiederum im Absperrzustand, vgl. Fig. 12.

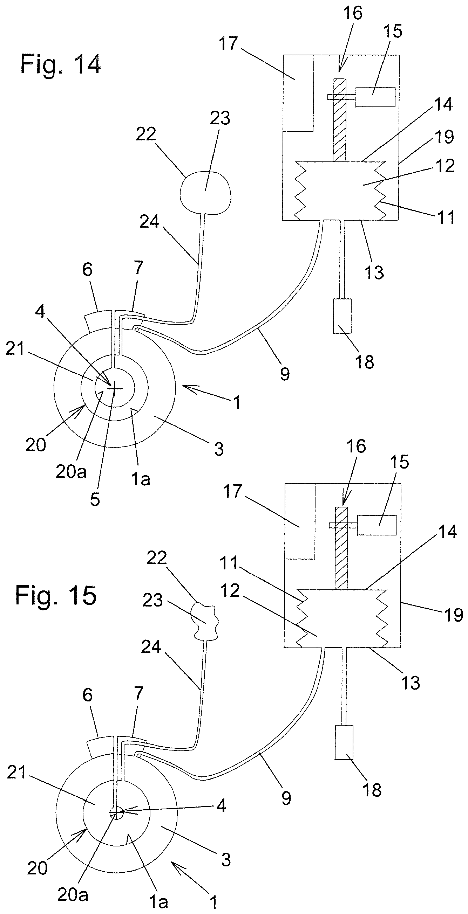

In den Fig. 14 und 15 ist ein zweites Ausführungsbeispiel einer medizinischen Einrichtung gemäß der Erfindung dargestellt. Der Aufbau der Pumpeinheit 10 und des Speicherbehälters 22 entspricht jenem der zuvor erläuterten medizinischen Einrichtungen. In den Erläuterungen zum zweiten Ausführungsbeispiel wird hierbei hauptsächlich auf die Unterschiede zum ersten Ausführungsbeispiel gemäß der Erfindung hingewiesen. Abgesehen von den im Folgenden angeführten Unterschieden gelten die Erläuterungen zum ersten Ausführungsbeispiel auch für das zweite Ausführungsbeispiel.

Bei der medizinischen Einrichtung gemäß dem zweiten Ausführungsbeispiel ist vorgesehen, dass der Expansionskörper 20 an einer der Durchtrittsöffnung 4 zugewandten Seite des Bandteils 1, also am Innenabschnitt 1a am Bandteil 1 angeordnet ist. Dies ist in den Fig. 14 und 15 schematisch dargestellt, wobei sich die Expansionskammer 21 in Richtung der Längserstreckung des Bandteils 1 erstreckt, im Wesentlichen über die gesamte Länge des Bandteils 1. Der Expansionskörper 20 ist also im zweiten Ausführungsbeispiel schlauchartig ausgebildet, mit beidseitig verschlossenen Enden.

Der am Bandteil 1 angeordnete Expansionskörper 20 weist im geschlossenen Zustand des Bandteils 1 bezogen auf die Umfangsrichtung der Längsmittelachse 5 eine im Wesentlichen umlaufende Anlagefläche 20a zur Anlage am Körpergewebe 2 auf.

Im Absperrzustand des geschlossenen Bandteils 1 wird die Hohlkammer 3 mit einer solchen Menge von Arbeitsfluid befüllt, dass der Körperkanal geschlossen ist, vgl. Fig. 14. Der Druck des Arbeitsfluids entspricht dem vom Körpergewebe ausgeübten Gegendruck plus einer gegebenenfalls zu überwindenden elastischen Rückstellkraft des Bandteils 1.

Wenn Arbeitsfluid abgelassen wird, nimmt das Bandteil 1 den nicht gesondert dargestellten Freigabezustand ein, in welchem der Körperkanal geöffnet ist.

Steigt der Druck im Körperinneren (=Körperinnendruck) ausgehend vom in Fig. 14 dargestellten Absperrzustand des Bandteils 1 bei Vorliegen eines Stressereignisses an, so wirkt dieser Körperinnendruck auf den Speicherbehälter 22 ein. Ist der Körperinnendruck, und damit der Druck des Hilfsfluids, größer als der vom Körpergewebe ausgeübte Gegendruck auf den Expansionskörper 20, so wird der Speicherbehälter 22 komprimiert und Hilfsfluid aus der Speicherkammer 23 in die Expansionskammer 21 des Expansionskörpers 20 geleitet. Falls der Expansionskörper 20 der Expansion der Expansionskammer 21 eine elastische Rückstellkraft entgegensetzt, ist auch diese durch den Druck des Hilfsfluids zu überwinden. Durch die Vergrößerung des Volumens der Expansionskammer 21 wirkt der Expansionskörper 20 direkt komprimierend auf das den Körperkanal umgebenden Körpergewebe 2 ein, um ein zuverlässiges Absperren des Körperkanals während eines Stressereignisses zu gewährleisten, vgl. den in Fig. 15 dargestellten Stresszustand des Bandteils 1, bei welchem eine Zusatzkraft auf das durch die Durchtrittsöffnung 4 geführte Körpergewebe 2 aufgebracht ist. Das Körpergewebe 2 selbst ist in den Fig. 14 und 15 nicht eingezeichnet.

Erreicht der Körperinnendruck wieder einen Grundzustand, d.h. ohne dass ein Stressereignis vorliegt, strömt das Hilfsfluid wiederum aus der Expansionskammer 21 in den Hilfsfluidbehälter 22 zurück, vgl. den in Fig. 14 dargestellten Absperrzustand des Bandteils 1.

Das Zurückströmen des Hilfsfluids in den Speicherbehälter 22 erfolgt somit durch Einwirken des vom Körpergewebe 2 ausgeübten Gegendruckes zusammen mit dem von der mit Arbeitsfluid gefüllten Hohlkammer 3 auf den Expansionskörper 20 ausgeübten Druck. Falls der Expansionskörper 20 der Expansion der Expansionskammer 21 eine elastische Rückstellkraft entgegensetzt, kann diese beim Zurückströmen des Hilfsfluids unterstützend wirken.

Im zweiten Ausführungsbeispiel kann vorgesehen sein, dass der Expansionskörper 20 und das Bandteil 1 materialeinstückig ausgebildet sind. Günstigerweise ist vorgesehen, dass der Expansionskörper 20 elastisch dehnbar ausgebildet ist. In einer anderen Ausführungsform könnte aber auch vorgesehen sein, dass der Expansionskörper 20 im Wesentlichen undehnbar und/oder biegeschlaff ausgebildet ist.

Abgesehen von der in den Ausführungsbeispielen gezeigten Pumpeinheit könnte der Speicherbehälter grundsätzlich auch in Kombination mit einer im Stand der Technik hinlänglich bekannten, beispielsweise manuelle betätigbaren Pumpeinheit verwendet werden.

**Legende zu den Hinweisziffern:**

| | | | |
|---|---|---|---|
| 1 | Bandteil | 12 | Innenraum |
| 1a | Innenabschnitt | 13 | Bodenteil |
| 1b | Rückenabschnitt | 14 | Stellelement |
| 2 | Körpergewebe | 15 | Antrieb |
| 3 | Hohlkammer | 16 | Getriebe |
| 4 | Durchtrittsöffnung | 17 | Steuerelektronik |
| 5 | Längsmittelachse | 18 | Port |
| 6 | erstes Verschlussteil | 19 | Gehäuse |
| 6a | Einstecköffnung | 20 | Expansionskörper |
| 7 | zweites Verschlussteil | 20a | Anlagefläche |
| 7a | Zunge | 21 | Expansionskammer |
| 8 | Anschlussstutzen | 22 | Speicherbehälter |
| 9 | Arbeitsfluidleitung | 22a | Wand |
| 9', 9" | Abschnitt der Arbeitsfluidleitung | 22b | Armierung |
| | | 23 | Speicherkammer |
| 10 | Pumpeinheit | 24 | Hilfsfluidleitung |
| 11 | Pumpteil | 25 | Verbindungsleitung |

## Patentansprüche

1. Medizinische Einrichtung zum Absperren eines Körperkanals, umfassend
- ein Bandteil (1), das um das den Körperkanal umgebende Körpergewebe (2) legbar ist und zu einem eine Durchtrittsöffnung (4) für das Körpergewebe (2) umschließenden Ring schließbar ist und das eine Hohlkammer (3) aufweist, die einen Teil eines Arbeitsfluid-Aufnahmeraums der Einrichtung zur Aufnahme von Arbeitsfluid darstellt, und
- eine Pumpeinheit (10) zum Fördern des Arbeitsfluids, wobei durch Einbringen des Arbeitsfluids in die Hohlkammer (3) die Durchtrittsöffnung (4) verkleinerbar ist, und
- einen Speicherbehälter (22) mit einer eine Speicherkammer (23) begrenzenden flexiblen Wand (22a), wobei zum Aufbringen einer Zusatzkraft auf das durch die Durchtrittsöffnung (4) geführte Körpergewebe (2) das Volumen der Speicherkammer (23) durch eine Erhöhung eines auf den Speicherbehälter (22) einwirkenden Umgebungsdrucks verkleinerbar ist, und die Speicherkammer (23) einen Teil eines Hilfsfluid-Aufnahmeraums der Einrichtung zur Aufnahme eines vom Arbeitsfluid getrennten Hilfsfluids bildet und der Hilfsfluid-Aufnahmeraum im Weiteren eine Expansionskammer (21) eines Expansionskörpers (20) der Einrichtung umfasst, wobei die Expansionskammer (21) zum Aufbringen der Zusatzkraft mit dem Hilfsfluid befüllbar ist,
**dadurch gekennzeichnet, dass** die Wand (22a) des Speicherbehälters (22) zumindest im Wesentlichen undehnbar ausgebildet ist.

2. Medizinische Einrichtung nach Anspruch 1, **dadurch gekennzeichnet, dass** der Expansionskörper (20) im Arbeitsfluid-Aufnahmeraum angeordnet ist.

3. Medizinische Einrichtung nach Anspruch 1, **dadurch gekennzeichnet, dass** der Expansionskörper (20) an einer der Durchtrittsöffnung (4) zugewandten Seite des Bandteils (1) am Bandteil (1) angeordnet ist.

4. Medizinische Einrichtung nach einem der Ansprüche 1 bis 3, **dadurch gekennzeichnet**, **dadurch gekennzeichnet, dass** das Volumen der Speicherkammer (23) ausgehend von einem maximal entfalteten Zustand des Speicherbehälters (22) bei einer Erhöhung des Drucks in der Speicherkammer (23) um 0,1 bar um weniger als 10%, vorzugsweise um weniger als 5%, zunimmt.

5. Medizinische Einrichtung nach einem der Ansprüche 1 bis 4, **dadurch gekennzeichnet, dass** die Wand (22a) des Speicherbehälters (22) eine zumindest im Wesentlichen undehnbare Armierung aufweist.

6. Medizinische Einrichtung nach einem der Ansprüche 1 bis 5, **dadurch gekennzeichnet, dass** die Wand (22a) des Speicherbehälters (22) eine Lage aus einer zumindest im Wesentlichen undehnbaren Kunststofffolie aufweist oder daraus besteht.

## Claims

1. A medical device for shutting off an anatomical channel, comprising
- a band part (1) which can be placed around the body tissue (2) surrounding the anatomical channel and which can be closed to form a ring that encloses a through-opening (4) for the body tissue (2), and which has a hollow chamber (3) constituting a part of a working fluid receiving space of the device for receiving working fluid, and
- a pump unit (10) which serves to convey the working fluid, wherein the through-opening (4) can be made smaller by introducing the working fluid into the hollow chamber (3), and
- a storage container (22) with a flexible wall (22a) delimiting a storage chamber (23), wherein, in order to apply an additional force to the body tissue (2) guided through the through-opening (4), the volume of the storage chamber (23) can be made smaller by increasing an ambient pressure acting on the storage container (22), and the storage chamber (23) forms a part of an auxiliary fluid receiving space of the device for receiving an auxiliary fluid separate from the working fluid, and the auxiliary fluid receiving space moreover comprises an expansion chamber (21) of an expansion body (20) of the device, wherein the expansion chamber (21) can be filled with the auxiliary fluid in order to apply the additional force
**characterized in that** the wall (22a) of the storage container (22) is designed to be at least substantially non-extensible.

2. The medical device according to claim 1, **characterized in that** the expansion body (20) is arranged in the working fluid receiving space.

3. The medical device according to claim 1, **characterized in that** the expansion body (20) is arranged at the band part (1), on a side of the band part (1) directed toward the through-opening (4).

4. The medical device according to one of claims 1 to 3, **characterized in that**, starting from a state of maximum deployment of the storage container (22), the volume of the storage chamber (23) increases by less than 10%, preferably by less than 5%, when the pressure in the storage chamber (23) increases by 0.1 bar.

5. The medical device according to one of claims 1 to 4, **characterized in that** the wall (22a) of the storage container (22) has an at least substantially non-extensible reinforcement.

6. The medical device according to one of claims 1 to 5, **characterized in that** the wall (22a) of the storage container (22) has or is composed of a layer of an at least substantially non-extensible plastic film.

## Revendications

1. Installation médicale pour bloquer un canal corporel comprenant
- un élément de bande (1) qui se place autour du tissus corporel (2) entourant le canal corporel, qui se ferme en forme d'anneau entourant un orifice de passage (4) pour le tissus corporel (2) et qui a une chambre creuse (3) constituant une partie d'une chambre réceptrice de fluide actif de l'installation recevant le fluide actif et
- une unité de pompe (10) pour transférer le fluide actif dont l'introduction dans la chambre creuse (3) réduit l'orifice de passage (4) et
- un réservoir (22) avec une paroi flexible (22a) délimitant une chambre accumulatrice (23), et pour appliquer une force supplémentaire sur le tissus corporel (2) passant dans l'orifice (4), on réduit le volume de la chambre accumulatrice (23) en augmentant la pression ambiante agissant sur le réservoir (22), la chambre accumulatrice (23) formant une partie d'une chambre de réception d'un fluide auxiliaire de l'installation de réception d'un fluide auxiliaire, distinct du fluide actif et la chambre de réception du fluide auxiliaire comprenant en outre une chambre d'expansion (21) d'un corps expansible (20) de l'installation, la chambre expansible (21) étant destinée à être remplie avec le fluide auxiliaire pour appliquer la force complémentaire,
installation **caractérisée en ce que**
la paroi (22a) du réservoir (22) est au moins pratiquement inextensible.

2. Installation médicale selon la revendication 1
**caractérisée en ce que**
le corps extensible (20) est prévu dans la chambre de réception de fluide actif.

3. Installation médicale selon la revendication 1
**caractérisée en ce que**
le corps expansible (20) se trouve dans l'élément de bande (20) sur le côté de l'élément de bande (20) tourné vers l'orifice de passage (4).

4. Installation médicale selon l'une des revendications 1 à 3 **caractérisée en ce que**
le volume de la chambre accumulatrice (23) augmente moins de 10%, de préférence moins de 5% par rapport à l'état de déploiement maximum du réservoir (22) pour une augmentation de la pression dans la chambre accumulatrice (23) de 0, 1 bar.

5. Dispositif médical selon l'une des revendications 1 à 4 **caractérisée en ce que**
La paroi (22a) du réservoir (22) comporte une armature au moins pratiquement inextensible.

6. Installation médicale selon l'une des revendications là 5 **caractérisée en ce que**
la paroi (22a) du réservoir (22) comporte une couche d'un film de matière plastique au moins pratiquement inextensible ou encore qui est réalisée avec un tel film.
